# EUROPEAN PATENT APPLICATION

(11) **EP 3 312 717 A1**
(43) Date of publication of application: **25.04.2018**
(21) Application number: 17181649.9
(22) Date of filing: 17.07.2017
(51) Int. Cl.: G06F 3/147, G06F 3/01, G09G 5/00

(54) **USER DEVICE AND COMPUTER PROGRAM STORED IN COMPUTER-READABLE MEDIUM FOR CONTROLLING DISPLAY**

(30) Priority: 19.10.2016 KR 20160135635
(71) Applicant: PIXEL Display Inc., Seoul 05719 (KR)
(72) Inventor: KWON, Tae Hyeon, 21124 Incheon (KR); LEE, Hyun Ho, 11949 Gyeonggi-do (KR)
(74) Representative: Zardi, Marco

(57) **Abstract**

Disclosed herein are a user device and a computer program stored in a computer-readable medium for controlling display. The user device includes a computer program stored in a computer-readable storage medium, and the computer program is executable by at least one processor and includes instructions adapted to cause the at least one processor to perform operations. The operations include the operations of: acquiring eye examination information of a user; determining display optimization information for the user at least partially based on user information including the eye examination information; and determining to adjust display settings of at least one user-related user device at least partially based on the determined display optimization information.

## Description

### BACKGROUND

### 1. Technical Field

The present disclosure relates to display technology, and more particularly to a user device and a computer program stored in a computer-readable medium for controlling display.

### 2. Description of the Related Art

Recently, the popularization of user devices, such as smartphones, tablet personal computers (PCs), etc., the construction of information technology (IT) infrastructures have proliferated rapidly. In general, the display units of user devices display various types of text and images, and thus users are exposed to the displays of user devices regardless of time and location. Accordingly, the vision of people today has been gradually declining.

Korean Patent No. 10-02532110 discloses a method and apparatus for controlling the display of a screen.

Meanwhile, a user with low vision, a user with astigmatism in which an eye cannot focus on a single point, a user with nearsightedness in which a focus is formed in front of a retina when light is inappropriately refracted due to the abnormality of the cornea, crystalline lens or the like of an eye, a user with farsightedness in which a focus is formed behind a retina, etc. require a series of processes for setting a display unit in order to optimize the display of a user device. For example, the adjustment of font size, the adjustment of screen brightness, or color correction may be required. It will be apparent that the series of processes for setting a display unit is not necessarily performed only for users having ocular health problems, such as low vision.

According to statistics, the average period during which humans view display screens is about 400 minutes per day over the world. When cases where the eyes of users are unintentionally exposed to display screens are included, it is estimated that the average period during which humans view display screens is higher than the above period.

With the expansion of IT infrastructures and the popularization of user devices including display units, the number of people who wear glasses has also increased. Furthermore, the number of patients with dry eyes has increased two or more times over the past decade. In particular, over the past five years, the number of patients with glaucoma has recently increased two or more times among people in their teens or twenties who are frequently exposed to display screens, such as the display screens of PCs, smartphones, etc.

Therefore, there is a demand for the provision of a display environment optimized for the ocular state of a user.

### SUMMARY

The present invention has been conceived to overcome the above-described disadvantages, and an object of the present disclosure is to provide display optimized for a user.

According to an aspect of the present invention, there is provided a user device including a computer program stored in a computer-readable storage medium, the computer program being executable by at least one processor and including instructions adapted to cause the at least one processor to perform operations, the operations including the operations of: acquiring eye examination information of a user; determining display optimization information for the user at least partially based on user information including the eye examination information; and determining to adjust display settings of at least one user-related user device at least partially based on the determined display optimization information.

The operation of acquiring eye examination information of a user may include the operation of generating the eye examination information of the user at least partially based on photographed ocular data of the user acquired via a photographing module.

The photographed ocular data of the user may be acquired for the identity authentication of the user.

The operation of acquiring eye examination information of a user may include the operation of providing an eye examination interface including at least one of an image and text used for performance of an eye examination of the user and generating the eye examination information of the user at least partially based on responses of the user to the eye examination interface.

The eye examination information may include at least one of a plurality of pieces of ocular health information, including vision information, nearsightedness information, astigmatism information, amblyopia information, color weakness information, and risk information.

The eye examination information may include at least one of information about whether the user wears a correction means and information about a corrected ocular state of the user when the user wears the correction means.

The display optimization information may further include at least one of font type information, font size information, font brightness information, font contrast information, font-to-screen ratio information, screen brightness information, image size information, image brightness information, resolution information, and color correction information.

The operation of determining to adjust display settings may include the operations of: acquiring user device information, external environment information, and user preference information; and determining content display setting values based on the user device information, the external environment information, and the user preference information.

The user device information may include at least one of a pixel dimension, a pixel interval, display width, display height, display reflectance, an emission spectrum, resolution, a luminance value, and a type of user device; the external environment information may include at least one of a distance between an eye of the user and a display, surrounding brightness, and information about physical locations of the user and the user device; and the preference information may include at least one of acuity preferred by the user, brightness preferred by the user, and information about user feedback regarding previously corrected content.

The operations may further include the operations of: acquiring content to be displayed, wherein the content includes at least one of an image, text, and a shape; generating content corrected based on at least logic predetermined for correction of the content to be displayed; and determining to display the corrected content.

The operation of generating corrected content may include at least one of the operations of: correcting the image included in the content to be displayed; correcting the text included in the content to be displayed; and correcting the shape included in the content to be displayed.

According to another aspect of the present invention, there is provided a user device, including: an eye examination information acquisition unit configured to acquire eye examination information of a user; a display optimization information determination unit configured to determine display optimization information for the user at least partially based on user information including the eye examination information; and a display setting adjustment unit configured to determine to control display settings of at least one user-related user device at least partially based on the determined display optimization information.

The user device may further include a camera unit configured to acquire photographed ocular data of the user.

According to still another aspect of the present invention, there is provided a user device including at least one display unit, the user device including: a control unit configured to determine to adjust display settings at least partially based on display optimization information for a user, wherein the display optimization information is determined at least partially based on the user information including eye examination information of the user.

According to still another aspect of the present invention, there is provided a server, including a control module configured to determine display optimization information for a user at least partially based on user information including the eye examination information of the user, and to determine to adjust the display settings of at least one user-related user device at least partially based on the determined display optimization information.

### BRIEF DESCRIPTION OF THE DRAWINGS

The above and other objects, features and advantages of the present disclosure will be more clearly understood from the following detailed description taken in conjunction with the accompanying drawings, in which:
FIG. 1 is a diagram showing a system in which various embodiments of the present disclosure may be implemented;
FIG. 2 is a flowchart showing a method of controlling display according to embodiments of the present disclosure;
FIG. 3 is a block diagram showing a user device for controlling display according to embodiments of the present disclosure;
FIG. 4 is a flowchart showing a method of controlling display according to embodiments of the present disclosure;
FIG. 5 is a block diagram showing a user device for correcting display according to embodiments of the present disclosure;
FIGS. 6 and 7 are diagrams showing display optimized for a user according to embodiments of the present disclosure;
FIG. 8 is a diagram showing examples in which at least one user device is optimized based on the eye examination information of a user according to embodiments of the present disclosure; and
FIGS. 9 to 12 are diagrams showing user interfaces configured to be displayed on a user device according to embodiments of the present disclosure.

### DETAILED DESCRIPTION

Various embodiments will be described with reference to the accompanying drawings below. In the present specification, various descriptions are presented to provide the understanding of the present disclosure. However, it will be apparent that these embodiments can be practiced without requiring the specific descriptions. In the embodiments, well-known components are provided in the form of block diagrams in order to facilitate descriptions of the embodiments.

The terms "component," "module," and "system" used herein refer to a computer-related entity, hardware, firmware, software, the combination of hardware and software, or software in execution. For example, the term "component" may be, but is not limited to, a process running on a processor, a processor, an object, an execution thread, a program, and/or a computer. By way of example, both an application running on a computing device and the computing device may be a component. One or more components may reside within a process and/or an execution thread, and a component may be localized on one computer and/or distributed across two or more computers. In addition, these components may be executed from various computer readable media having various data structures stored thereon. The components may communicate by way of local and/or remote processes, for example, in accordance with signals having one or more data packets (e.g., data from one component interacting with another component in a local system, a distributed system, and/or across a network, such as the Internet).

The term "or" used herein is intended to refer to an inclusive "or" rather than an exclusive "or". That is, unless specified otherwise or clear from the context, "X uses A or B" is intended to refer to any of natural inclusive permutations. That is, if X uses A, X uses B, or X uses both A and B, "X uses A or B" is satisfied in any one of the foregoing examples. Furthermore, it should be understood that the term "and/or" used herein refers to and includes any and all combinations of one or more of associated listed items.

It should be understood that the terms "include" and/or "including" used herein specify the presence of stated features, integers, steps, operations, elements, and/or components, but do not preclude the presence or addition of one or more other features, integers, steps, operations, elements, components, and/or groups thereof. The singular forms "a," "an," and "the" are intended to include the plural forms as well, unless the context clearly indicates otherwise.

The terms "information" and "data" used herein may be often used to be interchangeable with each other.

The description herein is presented to enable those skilled in the art to use and practice the invention. Various modifications to the disclosed embodiments will be readily apparent to those skilled in the art and the general principles defined herein may be applied to other embodiments and applications without departing from the spirit and scope of the present invention. Accordingly, the present invention is not intended to be limited to the disclosed embodiments, but should be interpreted in the broadest sense consistent with the principles and features disclosed herein.

Embodiments of the present disclosure will be described in detail with reference to the accompanying drawings below.

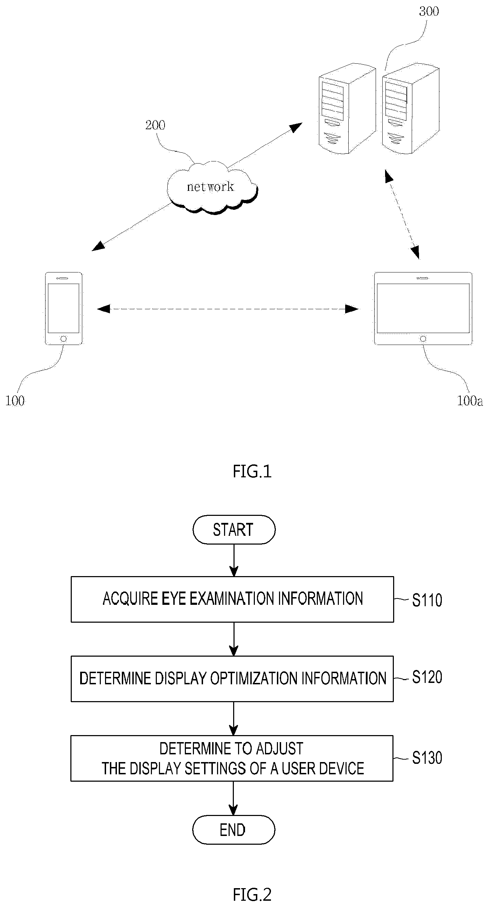

FIG. 1 is a diagram showing a system in which various embodiments of the present disclosure may be implemented.

Recently, with the rapid development of IT technology, the popularization of user devices including display units (e.g., notebook computers, smartphones, tablet PCs, etc.) has increased. Accordingly, the number of user devices, including display units, possessed by each individual has also increased.

According to statistics, the average period during which humans view display screens is about 400 minutes per day over the world. When cases where the eyes of users are unintentionally exposed to display screens are included, it is estimated that the average period during which humans view display screens is higher than the above period.

Accordingly, the number of people who wear glasses has also increased. Furthermore, the number of patients with dry eyes has increased two or more times over the past decade. In particular, over the past five years, the number of patients with glaucoma has recently increased two or more times among people in their teens or twenties who are frequently exposed to display screens, such as the display screens of PCs, smartphones, etc.

A method of providing display optimized for a user based on the eye examination information of the user according to embodiments of the present disclosure is disclosed.

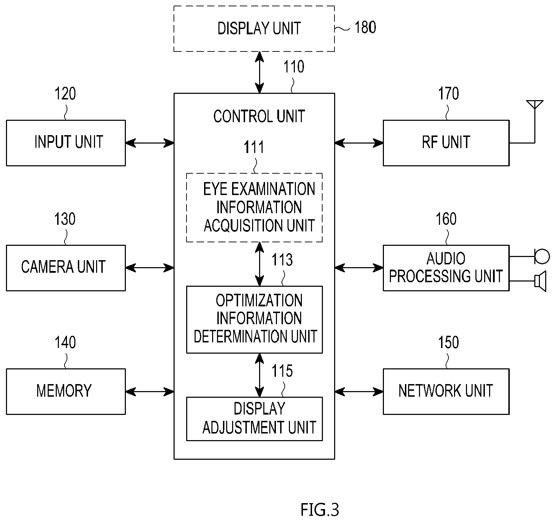

In FIGS. 2 and 3, a method of controlling the display of a user device is described.

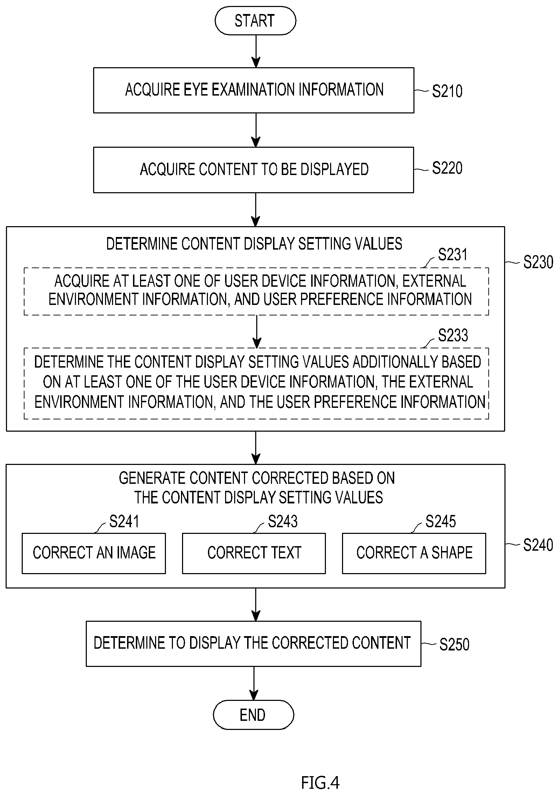

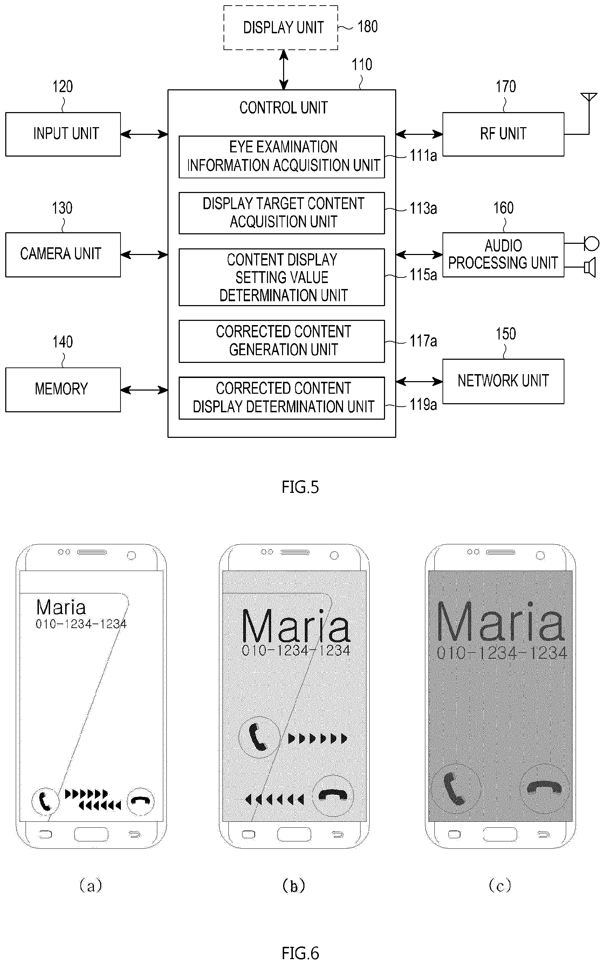

In FIGS. 4 and 5, a method of correcting content to be displayed on a user device is described.

Referring to FIG. 1, the system according to the embodiments of the present disclosure may include a user device 100, a network 200, and a server 300. The user device 100 and/or the server 300 according to the embodiments of the present disclosure may exchange data for the system according to the embodiments of the present disclosure with each other over the network 200.

The user device 100 in FIG. 1 may be referred to as a user device for controlling display in FIGS. 2 and 3.

The user device 100 according to the embodiments of the present disclosure may acquire the eye examination information of the user. The user device 100 according to the embodiments of the present disclosure may determine display optimization information for the user at least partially based on user information including the eye examination information. Furthermore, the user device 100 may determine to adjust the display settings of a user device at least partially based on the determined display optimization information. Moreover, the user device 100 may determine to adjust the display settings of at least one user-related user device 100a at least partially based on the determined display optimization information. In connection with this, a description will be given with reference to FIG. 2 below.

In this case, the at least one user-related user device 100a may refer to another user device possessed by the user. As another example, the at least one user-related user device 100a may refer to another user device with which an account of the user is shared. In this case, the at least one user-related user device 100a may include at least one display unit.

The user device 100 according to the embodiments of the present disclosure may refer to any device capable of using a wireless connection mechanism, such as an electronic communication device, a TV, a navigation system, a camcorder, a camera, a user terminal, user equipment, a mobile device, a PC capable of wireless communication, a mobile phone, a kiosk, a cellular phone, a cellular device, a cellular terminal, a subscriber unit, a subscriber station, a mobile station, a terminal, a remote station, a PDA, a remote terminal, an access terminal, a user agent, a portable device having a wireless connection function, or a wireless modem, but is not limited thereto. Furthermore, the user device 100 may refer to any device capable of using a wired connection mechanism, such as a wired fax, a PC including a wired modem, a wired phone, or a terminal capable of wired communication, but is not limited thereto. For example, the user device 100 according to the embodiments of the present disclosure may be a remote control configured to implement the system according to the embodiments of the present disclosure.

The user device 100 may transmit and/or receive various types of information to and/or from the server 300 and/or the at least one user-related user device 100a via a web browser or mobile application.

The network 200 according to the embodiments of the present disclosure may use various wired communication systems, such as a Public Switched Telephone Network (PSTN) system, an x Digital Subscriber Line (xDSL) system, a Rate Adaptive DSL (RADSL) system, a multi rate DSL (MDSL) system, a Very High Speed DSL (VDSL) system, a Universal Asymmetric DSL (UADSL) system, a High Bit Rate DSL (HDSL) system, a Local Area Network (LAN) system, etc.

Furthermore, the network 200 presented herein may use various wireless communication systems, such as a Code Division Multi Access (CDMA) system, a Time Division Multi Access (TDMA) system, a Frequency Division Multi Access (FDMA) system, an Orthogonal Frequency Division Multi Access (OFDMA) system, a Single Carrier-FDMA (SC-FDMA) system, and other systems.

The network according to one embodiment of the present disclosure may be composed of various types of networks, such as a wired network, a wireless network, etc., and may be composed of various communication networks, such as a Personal Area Network (PAN), a Wide Area Network (WAN), etc. Furthermore, the network may be the well-known World Wide Web (WWW), and may use a wireless transmission technology used for short range communication, such as Infrared Data Association (IrDA) or Bluetooth.

The technologies described herein may be used in not only the above-described networks but also other networks.

The server 300 according to the embodiments of the present disclosure may include a control module configured to determine the display optimization information for the user at least partially based on the user information including the eye examination information of the user and to determine to adjust the display settings of the at least one user-related user device at least partially based on the determined display optimization information.

The server 300 according to the embodiments of the present disclosure may receive the eye examination information of the user from the user device 100. Alternatively, the server 300 may receive the eye examination information of the user from another server (not shown; for example, a hospital server, an optician's shop server, or the like).

Since details of the server 300 according to the embodiments of the present disclosure correspond to a method performed in the user device 100, a detailed description thereof is omitted below.

In other embodiments, the user device 100 in FIG. 1 may be referred to as a user device for correcting display in FIGS. 3 and 4.

Referring to FIG. 1 again, the system according to embodiments of the present disclosure may include the user device 100, the network 200, and the server 300. The user device 100 and the server 300 according to the embodiments of the present disclosure may exchange data for the system according to the embodiments of the present disclosure with each other over the network 200.

The user device 100 in FIG. 1 may be referred to as a user device for displaying content optimized for the user based on the eye examination information of the user in FIGS. 4 and 5.

According to the embodiments of the present disclosure, the user device 100 may include at least one display unit. The user device 100 including the at least one display unit may acquire the eye examination information of the user. In this case, the eye examination information includes at least one of vision information, nearsightedness information, astigmatism information, presbyopia information, and farsightedness information, but the range of rights of the present disclosure is not limited thereto. For example, the eye examination information may include at least one of corneal morphological information (including at least one of corneal curvature information, ametropic information, pupil size information, interpupillary distance information, intraocular pressure information, corneal structure information, corneal thickness information, and corneal shape and symmetry information), visual field examination result information, retina information acquired by retinography, stiffness information, optic nerve layer thickness information, information about the number and shape of corneal endothelial cells, visual sensitivity, detailed sensitivity, and acuity.

Furthermore, the user device 100 may acquire content to be displayed. The user device 100 according to the embodiments of the present disclosure may determine content display setting values. The content display setting values may be determined based on at least the above-described eye examination information and logic predetermined for the correction of the content to be displayed. According to the embodiments of the present disclosure, content corrected based on the determined content display setting values may be generated. The corrected content may be displayed on the display unit of the user device 100. In connection with this, a description will be given below with reference to FIGS. 4 and 5.

The user device 100 according to the embodiments of the present disclosure may refer to any device capable of using a wired connection mechanism, such as an electronic communication device, a TV, a navigation system, a camcorder, a camera, a user terminal, a user device, a mobile device, a PC capable of wireless communication, a mobile phone, a kiosk, a cellular phone, a cellular device, a cellular terminal, a subscriber unit, a subscriber station, a mobile station, a terminal, a remote station, a PDA, a remote terminal, an access terminal, a user agent, a portable device having a wireless connection function, or a wireless modem, but is not limited thereto. Furthermore, the user device 100 may refer to a wired fax, a PC including a wired modem, a wired phone, a terminal capable of wired communication, or the like, but is not limited thereto.

The user device 100 may transmit and/or receive various types of information to and/or from the server 300 and/or the at least one user-related user device 100a via a web browser or mobile application.

Since the network 200 according to the embodiments of the present disclosure corresponds to the above-described network 200, a detailed description thereof is omitted below.

The server 300 according to the embodiments of the present disclosure may include an eye examination information acquisition module configured to acquire the eye examination information of the user. Furthermore, the server 300 may include a content acquisition module configured to acquire content to be displayed on a user device. In this case, the content includes at least one of an image, text, and a shape. The server 300 according to the embodiments of the present disclosure may determine the content display setting values based on at least the eye examination information and the logic predetermined for the correction of the content to be displayed. For example, the server 300 may include a content display setting determination module, and may determine the above-described content display setting values via the content display setting determination module. Content corrected based on the determined content display setting values may be generated. Furthermore, the server 300 according to the embodiments of the present disclosure may include a corrected content provision module in order to provide the corrected content to the user device 100 so that the corrected content is displayed on the user device 100.

Since the components included in the system shown in FIG. 1 have been disclosed for illustrative purposes, only some of the components may be included in the system or one or more additional components other than the above-described components may be further included in the system.

For example, the at least one user-related user device 100a may be further included in the system according to the embodiments of the present disclosure. The at least one user-related user device 100a may be connected to the user device 100 and/or the server 300 over the network 200.

As described above, the term "at least one user-related user device" used herein may refer to another user device possessed by the user. The at least one user-related user device 100a may include a display unit. The at least one user-related user device 100a may be interchangeable with a second user device 100a below.

The user device 100a may refer to any device capable of using a wireless connection mechanism, such as an electronic communication device, a TV, a navigation system, a camcorder, a camera, a user terminal, a user device, a mobile device, a PC capable of wireless communication, a mobile phone, a kiosk, a cellular phone, a cellular device, a cellular terminal, a subscriber unit, a subscriber station, a mobile station, a terminal, a remote station, a PDA, a remote terminal, an access terminal, a user agent, a portable device having a wireless connection function, or a wireless modem, but the range of rights of the present disclosure is not limited thereto.

The second user device 100a may include a control unit configured to control display settings at least partially based on the display optimization information for the user. In this case, the display optimization information is determined at least partially based on the user information including the eye examination information of the user.

Additionally, the information transmitted and received in the system described above with reference to FIG. 1 may be stored in the database or computer-readable storage medium of the user device 100, the server 300, and the at least one user-related user device 100a. The storage medium may include all types of storage media in which a program and data are stored to be read via a computer system. In an embodiment of the present disclosure, the medium may include Read-Only Memory (ROM), Random Access Memory (RAM), Compact Disc (CD)-ROM, Digital video Disc (DVD)-ROM, magnetic tape, a floppy disk, an optical data storage device, etc. Additionally, the medium may be distributed across systems connected over a network, and may store computer-readable codes and/or instructions in a distributed manner.

FIG. 2 is a flowchart showing a method of controlling display according to embodiments of the present disclosure.

In an embodiment of the present disclosure, one or more of the steps shown in FIG. 2 may be omitted. Furthermore, since the steps described in FIG. 2 have been disclosed for illustrative purposes, one or more additional steps may be further included in the range of rights of the present disclosure.

The steps shown in FIG. 2 may be performed via the user device 100. In greater detail, for example, the following method may be performed via the modules of the user device 100.

As another example, the following steps may be performed via a computer-readable storage medium for the control of display. Furthermore, the method described in FIG. 2 may be performed via the hardware or OS of a server 300 (see FIG. 1) according to another embodiment of the present disclosure.

The above-described steps are described only for illustrative purposes according to embodiments of the present disclosure, and the method may further include one or more additional steps or may be performed through a smaller number of steps. Furthermore, some of the steps shown in FIG. 2 may be omitted if necessary, or one or more additional steps may be further included if necessary.

A case in which the steps described in FIG. 2 are performed via the user device 100 is described below.

According to embodiments of the present disclosure, eye examination information may be acquired at step S110.

In this case, the eye examination information may refer to information which is obtained by an examination performed to check whether vision is good or bad, color blindness, the abnormality of an eye, or the like. The eye examination information according to the embodiments of the present disclosure may include at least one of various types of ocular health information including vision information, nearsightedness information, astigmatism information, amblyopia information, color weakness information, and risk information, but the range of rights of the present disclosure is not limited thereto.

According to the embodiments of the present disclosure, for example, the eye examination information may be received from the server 300.

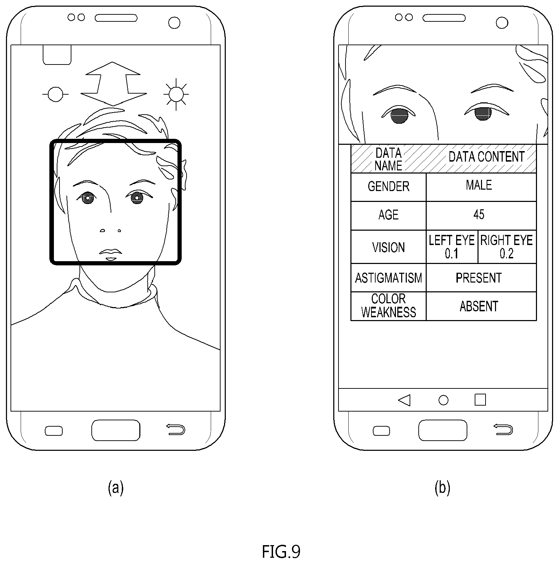

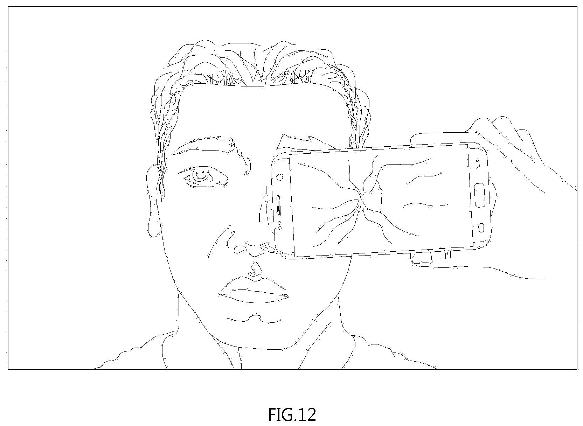

According to the embodiments of the present disclosure, the eye examination information may be generated at least partially based on the photographed ocular data of a user acquired via the photographing module of a user device 100 (e.g., a camera unit). For this, see FIG. 9. The photographed ocular data may be received from the server 300 or an external server (e.g., a hospital server, an optician's shop server, or the like). Alternatively, the photographed ocular data may be acquired for the identity authentication of the user. For this, see FIG. 12.

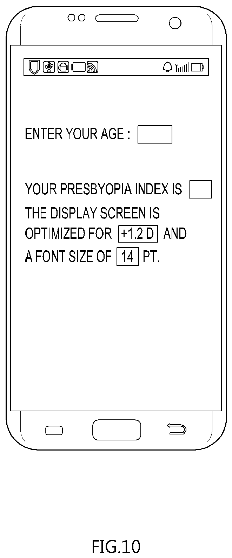

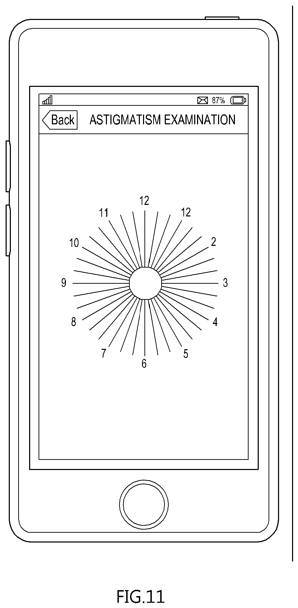

According to the embodiments of the present disclosure, the eye examination information may be generated at least partially based on responses of the user to an eye examination interface including at least one of an image and text used to perform the eye examination of the user. For this, see FIGS. 10 and 11.

The term "ocular health information" used herein may include "risk information," and may be determined through the analysis of the eye examination information, acquired from the user, at predetermined period intervals.

According to the embodiments of the present disclosure, display optimization information for the user may be determined at least partially based on user information including the eye examination information at step S120.

The display optimization information may include at least one of font type information, font size information, font brightness information, front contrast information, font-to-screen ratio information, screen brightness information, image size information, image brightness information, resolution information, and color correction information, but the range of rights of the present disclosure is not limited thereto.

The user information according to the embodiments of the present disclosure may include various types of information, e.g., user identification information, user age information, etc., in addition to the eye examination information, but the range of rights of the present disclosure is not limited thereto.

The display optimization information according to the embodiments of the present disclosure may be determined additionally based on at least one of user device attribute information, external environment information, and preference information.

In greater detail, for example, the user device attribute information may include resolution information, information about the type of user device, information about the size of the user device, and the like, but the range of rights of the present disclosure is not limited thereto. For example, the external environment information according to the embodiments of the present disclosure may include information about the physical locations of the user and the user device. Information about lighting around the user or the user device may be included in the external environment information. Optionally or alternatively, the external environment information may include weather information about the physical location of the user, but the range of rights of the present disclosure is not limited thereto.

The preference information according to the embodiments of the present disclosure may be information corresponding to user feedback regarding the display optimization information for the user, which is determined at least partially based on the user information including the eye examination information.

According to the embodiments of the present disclosure, the display settings of the user device may be determined to be adjusted at least partially based on the determined display optimization information at step S130.

According to the embodiments of the present disclosure, the display optimization information may be determined by considering the above-described various types of information regarding a plurality of factors. Accordingly, display optimized for the user may be provided.

According to the embodiments of the present disclosure, the settings of display may be automatically adjusted based on the display optimization information determined at least partially based on the user information including the eye examination information of the user without manual adjustment in order to optimize the settings of the display. Accordingly, the convenience of the user can be improved.

Additionally, the display settings of the at least one user-related user device may be determined to be adjusted at least partially based on the determined display optimization information. Accordingly, a user using a plurality of user devices does not need to adjust the display settings of each of the user devices, and thus the convenience of the user can be improved. In connection with this, a description will be given with reference to FIG. 5 below.

The above description has been given only for illustrative purposes according to the embodiments of the present disclosure, and thus various unmentioned examples may be included in the range of rights of the present disclosure.

FIG. 3 is a block diagram showing a user device 100 for controlling display according to embodiments of the present disclosure.

More specifically, referring to FIG. 3, the user device 100 according to the embodiments of the present disclosure may include a control unit 110, an input unit 120, a camera unit 130, memory 140, a network unit 150, an audio processing unit 160, an RF unit 170, and a display unit 180.

The components of the user device 100 shown in FIG. 3 are not essential, and thus the user device 100 having a larger or smaller number of components may be implemented.

The components will be described sequentially below.

The user device 100 according to the embodiments of the present disclosure may include the control unit 110. The control unit 110 may control and process various operations of the user device 100.

The control unit 110 according to the embodiments of the present disclosure may include an eye examination information acquisition unit 111, a display optimization information determination unit 113, and a display setting adjustment unit 115.

The eye examination information acquisition unit 111 according to the embodiments of the present disclosure may acquire the eye examination information of a user. The display optimization information determination unit 113 according to the embodiments of the present disclosure may determine display optimization information for the user at least partially based on user information including the eye examination information. The display setting adjustment unit 115 according to the embodiments of the present disclosure may determine to adjust the display settings of the user device at least partially based on the determined display optimization information. The display setting adjustment unit 115 may additionally determine to adjust the display settings of at least one user-related user device at least partially based on the determined display optimization information.

The user device 100 may generate the eye examination information of the user at least partially based on the photographed ocular data of the user acquired by a photographing module. The photographed ocular data may be received from a server 300 or an external server (e.g., a hospital server, an optician's shop server, or the like). Alternatively, the photographed ocular data may be acquired via the photographing module of the user device 100 (e.g., the camera unit 130). For this, see FIG. 9. The photographed ocular data of the user may be acquired for the identity authentication of the user. For this, see FIG. 12.

The user device 100 according to the embodiments of the present disclosure may provide an eye examination interface including at least one of an image and text used to perform the eye examination of the user. The eye examination information of the user may be generated at least partially based on responses of the user to the eye examination interface. For this, see FIGS. 10 and 11.

For example, the control unit 110 may include not only hardware, such as a central processing unit, a web server, or the like, but also software, such as an operating system, a control program, or the like.

For example, the input unit 120 according to the embodiments of the present disclosure may be a key input unit, but the range of rights of the present disclosure is not limited thereto. The input unit 120 includes keys configured to receive number and character information. According to the embodiments of the present disclosure, the input unit 120 may include a key configured to execute an application for the application of the system according to the embodiments of the present disclosure. Alternatively, the input unit 120 may include a key configured to initialize the display settings adjusted based on the display optimization information.

The camera unit 130 according to the embodiments of the present disclosure may be configured to acquire the photographed ocular data of the user. As described above, the eye examination information of the user may be generated at least partially based on the photographed ocular data of the user. The photographed ocular data of the user may be acquired for the identity authentication of the user. According to the method of controlling display according to the embodiments of the present disclosure, the eye examination information may be generated from the photographed ocular data acquired for the identity authentication without the intentional acquisition of photographed ocular data, and thus the convenience of the user can be improved.

The camera unit 130 according to the embodiments of the present disclosure may perform photographing to measure the vision of the user, and may include a camera sensor configured to convert an acquired optical signal into an electrical signal and a signal processing unit configured to convert an analog image signal acquired via the camera sensor into digital data.

According to the embodiments of the present disclosure, the camera unit 130 may further include, for example, a light-emitting means used to measure the vision of the user. Accordingly, the camera unit 130 may receive light emitted by the light-emitting means, may photograph an eye of the user, and may perform image processing related to the distance to a pupil and changes in the state of the eye through the pupil via the image processing unit, thereby determining the vision of the user. For this purpose, the user device 100 may include at least one of a proximity sensor, an infrared sensor, an RF sensor, a gyro sensor, and an ultrasonic sensor in order to measure the distance, but the range of rights of the present disclosure is not limited thereto.

According to the embodiments of the present disclosure, although not shown in the drawing, the user device 100 may further include an image processing unit configured to generate data adapted to display the image signal of the eye output via the camera unit 130,.

The memory 140 according to the embodiments of the present disclosure stores various types of information generated during the processing of the system according to the embodiments of the present disclosure. For example, the memory 140 may store the acquired eye examination information, the user information including the eye examination information, the display optimization information for the user, information about at least one user-related user device including at least one display unit, etc. In particular, various types of data and programs adapted to provide display, in which a font, resolution, and/or the like has been appropriately adjusted for the user in connection with the eye examination information, may be also stored in the memory 140. As another example, the eye examination information and distance-based display optimization matching information may be also stored in the memory 140.

In other words, the memory 140 may store a program used for the above-described operation of the control unit 110, and may temporarily or permanently store input and output data. The control unit 110 generally controls the overall operation of the user device 100. Furthermore, according to an embodiment of the present disclosure, the control unit 110 may communicate with all the above-described various components and all various components to be described below, and thus may systematically control the operations of the components.

The memory 140 may include at least one type of storage medium selected from among flash memory, a hard disk, a multimedia card, card-type memory (for example, SD or XD memory, or the like), random access memory (RAM), static random access memory (SRAM), read-only memory (ROM), electrically erasable programmable read-only memory (EEPROM), programmable read-only memory (PROM), magnetic memory, a magnetic disk, and an optical disk.

Referring to FIG. 2, although the memory 140 is provided inside the user device 100 in the present embodiments, the user device 100 may operate in conjunction with web storage configured to perform the storage function of the memory 140 over the Internet.

Furthermore, referring to FIG. 1 again, although the memory 140 is provided inside the user device 100 in the present embodiments, the user device 100 may operate in conjunction with memory configured to perform a storage function and provided inside the server 300 connected over the network 200.

Various types of information used for the above-described operations and operations to be described below may be transmitted and received to and from the server 300 and/or the at least one user-related user device 100a over the network unit 150.

The network unit 150 may include a wired/wireless Internet module for network connection as a network module. A wireless Internet technology for the wireless Internet module may include Wireless LAN (WLAN), Wi-Fi, Wireless broadband (Wibro), World Interoperability for Microwave Access (Wimax), High Speed Downlink Packet Access (HSDPA), etc. A wired Internet technology for the wired Internet module may include Digital Subscriber Line (XDSL), Fibers to the home (FTTH), Power Line Communication (PLC), etc. However, it will be apparent to those skilled in the art that the range of rights of the present disclosure is not limited thereto.

The network unit 150 according to the embodiments of the present disclosure may additionally include a short range communication module, and may exchange data with another user device which is located within a relatively short distance from the user device 100 and includes a short range communication module. A short range communication technology for the short range communication module may include Bluetooth, Radio Frequency Identification (RFID), Infrared Data Association (IrDA), Ultra Wideband (UWB), ZigBee, etc., but the range of rights of the present disclosure is not limited thereto.

The audio processing unit 160 according to the embodiments of the present disclosure may play back a reception audio signal input or output via the control unit 110, or may transmit a transmission or reception audio signal generated via a microphone. In other words, when a user interface is displayed on the display unit 180 in order to acquire the eye examination information of the user, user input to the user interface may be performed in the form of audio input.

The RF unit 170 according to the embodiments of the present disclosure may perform a wireless communication function. The RF unit 170 may include an RF transmitter configured to perform frequency up-conversion and amplification on a signal to be transmitted, and an RF receiver configured to perform low-noise amplification and frequency down-conversion on a received signal.

According to the embodiments of the present disclosure, although not shown in the drawing, the user device 100 may further include a data processing unit. The data processing unit may include a transmitter configured to encode and modulate a transmitted signal, a receiver configured to demodulate and decode a received signal.

The display unit 180 according to the embodiments of the present disclosure may output user interfaces.

The display unit 180 may include at least one of a liquid crystal display (LCD), a thin film transistor-liquid crystal display (TFT LCD), an organic light-emitting diode (OLED), a flexible display, and a 3D display. Of these displays, some display modules may be transparent-type display modules or light transmission-type display modules which allow the outside to be viewed therethrough. These are referred to as transparent display modules. A representative example of such a transparent display module is a transparent OLED (TOLED) or the like.

In an embodiment of the present disclosure, two or more display units may be present according to the implementation of the user device 100. For example, in the user device 100, a plurality of displays may be disposed on a single surface in a separate or integrated form, or may be disposed on different surfaces. For example, the display unit may include both or any one of a display user disposed on the upper end portion of the device 100 and a display disposed on the lower end portion of the device 100. However, the locations at which the above-described display unit 180 is disposed are merely examples, and the display unit may be disposed at various locations according to a need for design or a visual effect.

In an embodiment of the present disclosure, the display unit 180 may be composed of a touch screen implemented to receive the selection input of the user. The display unit 180 composed of the touch screen may include a touch sensor. The touch sensor may be configured to convert a change, such as pressure applied to a specific portion of the display unit 180 or capacitance generated in a specific portion of the display unit 180, into an electrical input signal. The touch sensor may be configured to detect not only a touch location and a touch area but also a touch pressure. When touch input is applied to the touch sensor, a signal(s) corresponding to the touch input is transferred to a touch controller. The touch controller processes the signal(s), and transmits corresponding data to the control unit 110. This enables the control unit 110 to recognize the portion of the display unit 180 which has been touched.

According to the above-described embodiments of the present disclosure, display settings for screens to be displayed on the display unit 180 may be determined to be adjusted at least partially based on display optimization information for the user determined at least partially based on user information including the eye examination information of the user.

Referring to FIG. 3, part or all of the various embodiments described herein may be implemented in a computer- or similar device-readable recording or storage medium by using, for example, software, hardware, or the combination thereof.

According to hardware implementation, the embodiments described herein may be implemented using at least one of application specific integrated circuits (ASICs), digital signal processors (DSPs), digital signal processing devices (DSPDs), programmable logic devices (PLDs), field programmable gate arrays (FPGAs), processors, controllers, microcontrollers, micro-processors, and other electrical units configured to perform functions. In some cases, the embodiments described herein may be implemented as the control unit 110 itself.

According to software implementation, the embodiments, such as procedures or functions, described herein may be implemented using separate software modules. Each of the software modules may perform at least one function or operation described herein. Software codes may be implemented as a software application written in an appropriate program language. The software codes may be stored in the memory 140, and may be executed by the control unit 110.

FIG. 4 is a flowchart showing a method of controlling display according to embodiments of the present disclosure.

According to an embodiment of the present disclosure, one or more of the steps shown in FIG. 4 may be omitted. Furthermore, the steps shown in FIG. 4 are described for illustrative purposes, and thus one or more additional steps may be further included in the range of rights of the present disclosure.

The steps shown in FIG. 4 may be performed by a user device 100. In greater detail, the following method may be performed by, for example, the individual modules of the user device 100.

As another example, the following steps may be performed by a computer-readable storage medium adapted for the control of display. Furthermore, according to another embodiment of the present disclosure, the method shown in FIG. 4 may be performed by the hardware or OS of the server 300 (see FIG. 1).

The above-described steps are described only for illustrative purposes according to embodiments of the present disclosure, and, thus, may further include one or more additional steps or may be performed through a smaller number of steps. Furthermore, one or more steps shown in FIG. 4 may be omitted if necessary, and one or more additional steps may be further included if necessary.

A case in which the method shown in FIG. 4 is performed by the user device 100 is described below.

According to the embodiments of the present disclosure, eye examination information may be acquired at step S210.

In this case, the eye examination information includes at least one of vision information, nearsightedness information, astigmatism information, presbyopia information, and farsightedness information, but the range of rights of the present disclosure is not limited thereto. For example, the eye examination information may include at least one of corneal morphological information (including at least one of corneal curvature information, ametropic information, pupil size information, interpupillary distance information, intraocular pressure information, corneal structure information, corneal thickness information, and corneal shape and symmetry information), visual field examination result information, retina information acquired by retinography, stiffness information, optic nerve layer thickness information, information about the number and shape of corneal endothelial cells, visual sensitivity, detailed sensitivity, and acuity.

The eye examination information may be determined at least partially based on the photographed ocular data of a user. Optionally or alternatively, the eye examination information may be determined based on responses of the user to a user interface provided for the determination of the eye examination information. For the user interface provided for the determination of the eye examination information, see FIGS. 10 and 11. Alternatively, the eye examination information may be input directly from the user device 100, or may be received in the form of data from the server 300 related to the user device 100. For example, the eye examination information may be received from the hospital server 300 of a hospital where the ocular state examination of the user has been performed. Optionally or alternatively, the eye examination information may be received from a user device of an examiner who has performed the ocular state examination of the user.

The eye examination information according to the embodiments of the present disclosure may further include at least one of information about whether the user wears a correction means and information about the corrected ocular state of the user when the user wears the correction means. For example, the information about the corrected ocular state may be information about the measurement result of the ocular state examination when the user wears the correction means. Alternatively, the information about the corrected ocular state may be information about a target ocular state which is estimated to be obtained by the correction means.

According to the embodiments of the present disclosure, the user device 100 may acquire content to be displayed at step S220.

In this case, the content may include at least one of an image, text, and a shape, but the range of rights of the present disclosure is not limited thereto.

According to the embodiments of the present disclosure, content display setting values may be determined at step S230.

The content display setting values may be determined based on at least logic predetermined for the correction of content to be displayed.

In this case, the predetermined logic may be related to at least one of the quantification of the difference between an original image and an image viewed to the user, one or more factors related to the eye examination information, and overall image quality recognized by the user. For example, the predetermined logic may be determined in connection with a change in the sensitivity of the receptors of an eye based on luminance.

The predetermined logic may be determined based on at least one of a conditional optimization algorithm based on a gradient descent method, a conditional optimization algorithm based on the van Cittert Zernike theorem, and a wiener deconvolution algorithm, but the range of rights of the present disclosure is not limited thereto. For example, the algorithm based on the van Cittert Zernike theorem may be adapted to have a restriction applied by the use of the Jansson scheme.

Step S230 of determining content display setting values according to the embodiments of the present disclosure may include step S231 of acquiring at least one of user device attribute information, external environment information, and user preference information, and step S233 of determining content display setting values additionally based on at least one of the user device attribute information, the external environment information, and the user preference information.

In this case, the user device attribute information may include at least one of a pixel dimension, a pixel interval, display width, display height, display reflectance, an emitted spectrum, resolution, a luminance value, and the type of user device, but the range of rights of the present disclosure is not limited thereto.

In this case, the external environment information may include at least one of the distance between an eye of the user and display, surrounding brightness, and information about the physical locations of the user and the user device, but the range of rights of the present disclosure is not limited thereto.

In this case, the preference information may include at least one of acuity preferred by the user, brightness preferred by the user, and information about user feedback regarding previously corrected content, but the range of rights of the present disclosure is not limited thereto.

The content display setting values determined according to the embodiments of the present disclosure may be provided to another user-related user device and a server. Accordingly, the user may receive the content display setting values even when the user uses the other user device, and thus content optimized and corrected for the user can be displayed.

Content corrected based on the content display setting values determined according to the embodiments of the present disclosure may be generated at step S240.

Step S240 of generating the corrected content may include at least one of step S241 of correcting an image included in content, step S243 of correcting text included in the content, and step S245 of correcting a shape included in the content.

When the information about user feedback regarding previously corrected content includes information about the fact that the user prefers text correction, step S243 of correcting text may be performed. For example, text may be corrected through the performance of rendering, such as anti-aliasing. Although step S240 of generating the corrected content is generally performed to include the overall correction of an image, text, and a shape included in the content, at least part of the image, the text, and the shape may be determined not to be corrected based on the user preference information.

The above description has been given only for illustrative purposes according to embodiments of the present disclosure, and thus various unmentioned examples may be included in the range of rights of the present disclosure.

FIG. 5 is a block diagram showing a user device 100 for correcting display according to embodiments of the present disclosure.

More specifically, referring to FIG. 5, the user device 100 according to the embodiments of the present disclosure may include a control unit 110, an input unit 120, a camera unit 130, memory 140, a network unit 150, an audio processing unit 160, an RF unit 170, and a display unit 180.

The components of the user device 100 shown in FIG. 5 are not essential, and thus the user device 100 having a larger or smaller number of components may be also implemented.

Since the input unit 120, the camera unit 130, the memory 140, the network unit 150, the audio processing unit 160, the RF unit 170, and the display unit 180 according to the embodiments of the present disclosure shown in FIG. 5 may correspond to the input unit 120, the camera unit 130, the memory 140, the network unit 150, the audio processing unit 160, the RF unit 170, and the display unit 180 shown and described in FIG. 3, detailed descriptions thereof are omitted below.

The components included in the user device 100 in order to display content optimized for a user based on the eye examination information of the user are described below.

The user device 100 according to the embodiments of the present disclosure may include the control unit 110. The control unit 110 may control and process various operations of the user device 100.

The control unit 110 according to the embodiments of the present disclosure may include an eye examination information acquisition unit 111a, a display target content acquisition unit 113a, a content display setting determination unit 115a, a corrected content generation unit 117a, and a display determination unit 119a.

The eye examination information acquisition unit 111a according to the embodiments of the present disclosure may acquire the eye examination information of the user. The eye examination information may include at least one of vision information, nearsightedness information, astigmatism information, presbyopia information, and farsightedness information. The eye examination information may be determined based on at least the photographed ocular data of the user, may be determined based on responses of the user to a user interface provided for the determination of the eye examination information, or may be received in the form of data from at least one user device and a server.

The display target content acquisition unit 113a according to the embodiments of the present disclosure may acquire content to be displayed. In this case, the content may include at least one of an image, text, and a shape.

The content display setting determination unit 115a according to the embodiments of the present disclosure may determine content display setting values based on at least logic predetermined for the correction of the content to be displayed.

The corrected content generation unit 117a according to the embodiments of the present disclosure may generate content corrected based on the determined content display setting values, and the corrected content may be determined to be displayed by the display determination unit 119a.

The memory 140 according to the embodiments of the present disclosure stores various types of information generated during the processing of the system according to the embodiments of the present disclosure. For example, the memory 140 may store a program used for the above-described operation of the control unit 110, and may temporarily or permanently store input and/or output data. The control unit 110 generally controls the overall operation of the user device 100. Furthermore, according to an embodiment of the present disclosure, the control unit 110 may communicate with all the above-describe various components and all various components to be described below, and thus may systematically control the operations of the components.

Referring to FIG. 5, part and/or all of the various embodiments described herein may be implemented within a computer- or similar device-readable recording medium by using, for example, software, hardware, or the combination thereof.

According to hardware implementation, the embodiments described herein may be implemented using at least one of application specific integrated circuits (ASICs), digital signal processors (DSPs), digital signal processing devices (DSPDs), programmable logic devices (PLDs), field programmable gate arrays (FPGAs), processors, controllers, microcontrollers, microprocessors, and other function performance electric units. In some cases, the embodiments described herein may be implemented as the control unit 110 itself.

According to software implementation, embodiments, such as processes and functions, described herein may be implemented as separate software modules. Each of the software modules may perform one or more functions and operations described herein. The software codes may be implemented as a software application written in an appropriate program language. The software codes may be stored in the memory 140, and may be executed by the control unit 110.

FIG. 6 is a diagram showing display optimized for the user according to embodiments of the present disclosure.

According to the embodiments of the present disclosure, display optimization information for the user may be determined at least partially based on user information including eye examination information. Furthermore, the display optimization information for the user may be determined based on at least one of user device attribute information, external environment information, and preference information.

For example, it is assumed that first, second and third users having different pieces of age information and the same eye examination information are present. For example, it is assumed that the age information of first user is twenties, the age information of the second user is thirties, and the age information of the third user is sixties. According to the embodiments of the present disclosure, the display settings of user devices related to the first user, the second user and the third user may be adjusted at least partially based on "different pieces of display optimization information" generated based on "different pieces of age information (i.e., different pieces of user information)."

In other words, FIG. 6(a) may show a user interface which is displayed on the user device of the first user, FIG. 6(b) may show a user interface which is displayed on the user device of the second user, and FIG. 6(c) may show a user interface which is displayed on the user device of the third user.

According to the embodiments of the present disclosure, display optimization information for the user may be determined partially based on the eye examination information of the user, and an appropriate display environment may be provided to the user. For example, FIGS. 6(a), 6(b) and 6(c) may show pieces of content in which the sizes of fonts have been corrected based on presbyopia indices generated based on the pieces of presbyopia information of the users.

As another example, in a case where the same user uses the user device 100 in a room where a light is turned off during the day and in a case where the same user uses the user device 100 in a room where a light is turned on during the night, different pieces of display optimization information may be also determined.

Referring to FIGS. 6(a) to 6(c), for example, FIG. 6(a) may show a user interface which is displayed on the display unit of the user device 100 in a room where a light is turned on during the day. FIG. 6(b) may show a user interface which is displayed on the display unit of the user device 100 in a room where a light is turned off during the day. Furthermore, FIG. 6(c) may show a user interface which is displayed on the display unit of the user device 100 in a room where a light is turned off during the night.

As another example, the brightness of a user interface displayed on a display unit may be controlled based on the light sensitivity of the user determined from preference information received from the user or eye examination information.

The above-described examples are merely examples according to the embodiments of the present disclosure, but the range of rights of the present disclosure is not limited thereto.

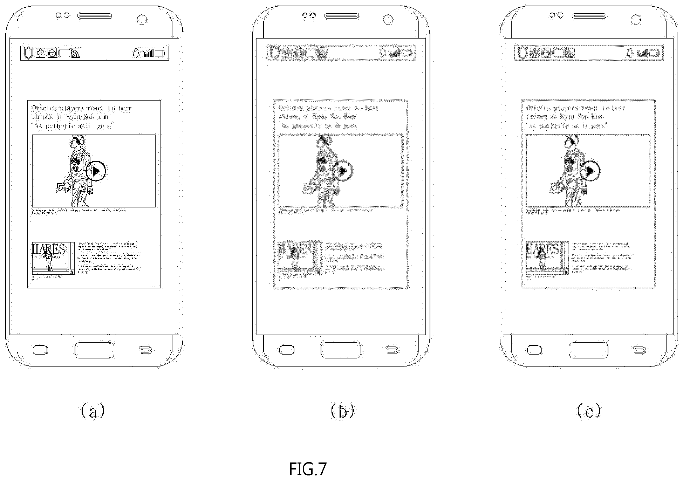

FIG. 7 are views showing display optimized for a user according to the embodiments of the present disclosure.

FIG. 7(a) shows an example of original content.

FIG. 7 (b) shows the form in which the original content is viewed to the user according to the eye examination state of the user.

FIG. 7(c) shows the form in which content corrected according to the embodiments of the present disclosure is viewed to the user.

FIGS. 7(a) to 7(c) show examples of the content displayed on the user device according to the embodiments of the present disclosure. According to the embodiments of the present disclosure, the user may be provided with optimized content displayed on the user device without requiring a separate correction means.

From the comparison between FIG. 7(b) and FIG. 7(c), it can be seen that the content provided to the same user has been improved. Preferably, according to the embodiments of the present disclosure, an improvement may be made such that FIG. 7(a) is the same as FIG. 7(c). For example, according to the embodiments of the present disclosure, FIG. 7 (b) may be corrected to FIG. 7(c) in connection with at least one of the quantification of the difference between the original image and the image viewed to the user, one or more factors related to the eye examination information, and overall image quality recognized by the user.

Furthermore, FIG. 7(b) may be corrected to correspond to FIG. 7(a) based on at least one of a gradient descent-based conditional optimization algorithm, a van Cittert Zernike-based conditional optimization algorithm, and a wiener deconvolution algorithm.

Furthermore, the term "content" used herein includes at least one of an image, text, and a shape. The content displayed on FIGS. 7(a) to 7(c) includes all of an image, text, and a shape. At least one of an image, text, and a shape may be corrected based on preference received from the user. For example, when only the correction of text and a shape is preferred, the image may be minimally corrected (or may not be corrected), and the text and the shape may be corrected based on user eye examination information. The above-described description corresponds merely to examples according to the embodiments of the present disclosure, and it will be apparent to those skilled in the art that the present disclosure is not limited thereto.

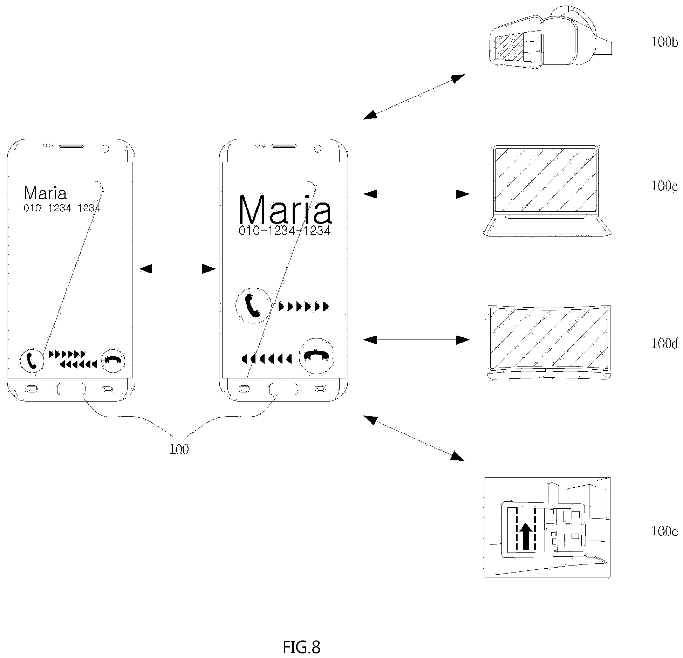

Referring to FIGS. 6 and 7, according to the above-described present disclosure, display can be optimized for the user. Furthermore, various types of information for display optimization may be provided to the user device and a server, thereby enabling the display settings of the at least one user-related user device to be also adjusted. For this, see FIG. 8.

FIG. 8 is a view showing the way that one or more user devices are optimized based on the eye examination information of a user according to embodiments of the present disclosure.

According to the embodiments of the present disclosure, the display settings of the user device 100 may be adjusted at least partially based on display optimization information which is determined at least partially based on user information including the eye examination information of the user.

As described in FIG. 8, the display settings of the at least one user-related user devices 100b, 100c, 100d and 100e may be also adjusted at least partially based on the display optimization information.

According to the embodiments of the present disclosure, content display setting values determined based on logic predetermined for the correction of content to be displayed may be provided to at least one of the at least one user-related user device and the server, thereby allowing content to be displayed on another user device to be corrected.

In other words, as shown in FIG. 8, content to be displayed on the at least one user-related user devices 100b, 100c, 100d and 100e may be allowed to be corrected.

With the recent popularization of IT devices, the number of users each possessing a plurality of user devices has increased. According to the embodiments of the present disclosure, each of the plurality of user devices of each of the users may be automatically optimized without requiring that the user manually optimize each of the plurality of user devices.

Referring to FIGS. 9 to 12, a user interface which is displayed on the user device 100 according to the embodiments of the present disclosure is now described. In this case, the user device 100 is described as a smartphone including at least one display unit, but the range of rights of the present disclosure is not limited thereto.

FIG. 9 shows user interfaces displayed on a user device in order to acquire eye examination information according to the embodiments of the present disclosure.

As described above, the eye examination information may be generated by photographing the eyes of the user via the photographing module of the user device 100 (e.g., the camera unit). Additionally, the eye examination information generated at least partially based on the photographed ocular data of the user acquired via the photographing module of the user device 100 may be displayed on the display unit 180. The eye examination information generated at least partially based on the photographed ocular data may be analyzed at predetermined period intervals, and may be processed into ocular health information including risk information. Moreover, the ocular health information may be provided to the server 300, and thus the server 300 may accumulate, record, and manage the eye examination information of the user.

FIGS. 10 and 11 show other user interfaces displayed on the user device in order to acquire eye examination information according to the embodiments of the present disclosure. The user interfaces shown in FIGS. 10 and 11 are merely examples of interfaces used for the determination of the vision and astigmatism of a user, but the range of rights of the present disclosure is not limited thereto.

According to the embodiments of the present disclosure, the eye examination information may be generated at least partially based on the responses of the user to an eye examination interface including at least one of an image and text used for the eye examination of the user. When the user interface is displayed on the display unit 180 in order to acquire the eye examination information of the user, the responses of the user may be implemented as, for example, voice inputs.

Furthermore, the user interface may be displayed on the display in order to receive the eye examination information from the user. For example, the user may input presbyopia age information via the user interface by referring to FIG. 10. Optionally or alternatively, the age input and presbyopia index in the user interface shown in FIG. 10 may be automatically input by referring to information previously received from the user (for example, at least one of previously input age information and previously generated eye examination information).

FIG. 12 shows a user interface which is displayed on a user device in order to authenticate a user according to the embodiments of the present disclosure.

Recently, as identity authentication methods, identify authentication methods using biometric identification information have been adopted in various technologies and/or devices. In this case, the identify authentication method using biometric identification information refer to an identity authentication method using biometric information regarding at least one of a signature, a face, an iris, a retina, a fingerprint, a voice, a hand shape, and hand blood vessels.

According to the embodiments of the present disclosure, the eye examination information of the user according to the embodiments of the present disclosure may be determined using photographed ocular data acquired to perform identity authentication required for the use of the user device 100. In other words, according to the method of controlling display according to the embodiments of the present disclosure, the eye examination information may be generated from the photographed ocular data acquired for identity authentication without intentionally acquiring the photographed ocular data, and thus the convenience of a user can be improved.

Furthermore, such photographed ocular data acquired for identity authentication required for the use of the user device 100 may be analyzed at predetermined period intervals. For example, analysis information including the ocular health information of the user may be provided to the user based on the photographed ocular data at the predetermined period intervals.

Additionally, the shown and above-described interfaces are merely examples adapted to implement the embodiments of the present disclosure in the user device 100, but the range of rights of the present disclosure is not limited thereto. In other words, the components displayed on the interfaces shown in FIGS. 9 to 12 and described above may be omitted, added or changed according to the need of a person skilled in the art and/or the user.

The above descriptions are merely examples of at least some effects according to the embodiments of the present disclosure, and it will be apparent to those skilled in the art that the effects according to the range of rights of the present disclosure are not limited by the above descriptions.

In one or more exemplary implementations, the described functions, steps, and operations may be implemented using hardware, software, firmware, or the combination thereof. In the case of software implementation, the functions may be stored in a computer-readable medium in the form of one or more instructions or codes, or may be transferred therethrough. The computer-readable medium includes a computer storage medium, and a communication medium including a medium configured to facilitate the transfer of a computer program from one location to another location. The computer storage medium may be any usable medium configured to be accessed by a general-purpose computer or a special-purpose computer. For example, the computer-readable medium may be RAM, ROM, EEPROM, CD-ROM, another optical disk storage medium, a magnetic disk storage medium, or another magnetic storage medium, or may be used to store a required program code means in the form of instructions or a data structure. Furthermore, the computer-readable medium may include another medium configured to be accessed by a general-purpose computer, a special-purpose computer, a general-purpose processor, or a special processor, but is not limited thereto. Furthermore, a connection means may be considered to be the computer-readable medium. For example, when software is transferred from a website, a server, or a remote source via a coaxial cable, an optical fiber cable, a stranded cable, or a Digital Subscriber Line (DSL) or by using a wireless technology, such as an infrared radio technology or microwave technology, an coaxial cable, an optical fiber cable, an stranded cable, a Digital Subscriber Line (DSL), or a wireless technology, such as an infrared radio technology or a microwave technology, may fall within the definition of the computer-readable medium. In this case, a disk used herein includes a Compact Disk (CD), a laser disk, an optical disk, a DVD, a floppy disk, or a Bluray disk. In this case, the disk may play back data in a magnetic manner, but may play back data in an optical manner by using laser. The combination thereof may be also included in the range of the computer-readable medium.

Those having ordinary knowledge in the art will appreciate that the above-described various exemplary logic blocks, modules, circuits, and algorithm steps may be implemented via electronic hardware, computer software, or the combination thereof. To make the compatibility between hardware and software clear, various exemplary elements, blocks, modules, circuits, and steps are described in terms of the functions thereof. Whether the functions are implemented via hardware or software is determined based on design restrictions applied to a specific application or an overall system. Those having ordinary knowledge in the art may implement the functions by using various methods for each specific application, but the implementation determination does not depart from the range of rights of the present disclosure.

Furthermore, various aspects or features described herein may be implemented as methods, devices, or products using standard programming and/or engineering techniques. Furthermore, the methods, the algorithm steps, and/or the operations described in connection with the aspects described herein may be directly implemented by hardware, a software module executed via a processor, or the combination thereof. Additionally, in some embodiments, the methods, the algorithm steps, or the operations may reside in the form of at least one of a machine-readable medium, codes stored in a computer-readable medium, a set of instructions, or the combination thereof, and may be integrated into a computer program product.

According to the above-described embodiments of the present disclosure, display optimized for a user can be provided.

The description herein is presented to enable those skilled in the art to use and practice the invention. Various modifications to the disclosed embodiments will be readily apparent to those skilled in the art and the general principles defined herein may be applied to other embodiments and applications without departing from the spirit and scope of the present invention. Accordingly, the present invention is not intended to be limited to the disclosed embodiments, but should be interpreted in the broadest sense consistent with the principles and features disclosed herein.

## Claims

1. A user device comprising a computer program stored in a computer-readable storage medium, the computer program being executable by at least one processor and including instructions adapted to cause the at least one processor to perform operations, the operations comprising the operations of:
acquiring eye examination information of a user;
determining display optimization information for the user at least partially based on user information including the eye examination information; and
determining to adjust display settings of at least one user-related user device at least partially based on the determined display optimization information;
wherein the display optimization information includes font brightness information, font contrast information, font-to-screen ratio information, screen brightness information, and color correction information; and
wherein the operation of determining to adjust display settings comprises the operations of:
acquiring user device information, external environment information, and user preference information; and
determining content display setting values based on the user device information, the external environment information, and the user preference information.

2. The user device of claim 1, wherein the operation of acquiring eye examination information of a user comprises the operation of generating the eye examination information of the user at least partially based on photographed ocular data of the user acquired via a photographing module.

3. The user device of claim 2, wherein the photographed ocular data of the user is acquired for identity authentication of the user.

4. The user device of claim 1, wherein the operation of acquiring eye examination information of a user comprises the operation of providing an eye examination interface including at least one of an image and text used for performance of an eye examination of the user and generating the eye examination information of the user at least partially based on responses of the user to the eye examination interface.

5. The user device of claim 1, wherein the eye examination information includes at least one of a plurality of pieces of ocular health information, including vision information, nearsightedness information, astigmatism information, amblyopia information, color weakness information, and risk information.

6. The user device of claim 1, wherein the eye examination information includes at least one of information about whether the user wears a correction means and information about a corrected ocular state of the user when the user wears the correction means.

7. The user device of claim 1, wherein the display optimization information further includes at least one of font type information, font size information, image size information, image brightness information, and resolution information.

8. The user device of claim 1, wherein:
the user device information includes at least one of a pixel dimension, a pixel interval, display width, display height, display reflectance, an emission spectrum, resolution, a luminance value, and a type of user device;
the external environment information includes at least one of a distance between an eye of the user and a display, surrounding brightness, and information about physical locations of the user and the user device; and
the preference information includes at least one of acuity preferred by the user, brightness preferred by the user, and information about user feedback regarding previously corrected content.

9. The user device of claim 1, wherein the operations further comprise the operations of:
acquiring content to be displayed, wherein the content includes at least one of an image, text, and a shape;
generating content corrected based on at least logic predetermined for correction of the content to be displayed; and
determining to display the corrected content.

10. The user device of claim 9, wherein the operation of generating corrected content comprises at least one of the operations of:
correcting the image included in the content to be displayed;
correcting the text included in the content to be displayed; and
correcting the shape included in the content to be displayed.

11. A user device, comprising:
an eye examination information acquisition unit configured to acquire eye examination information of a user;
a display optimization information determination unit configured to determine display optimization information for the user at least partially based on user information including the eye examination information; and
a display setting adjustment unit configured to determine to control display settings of at least one user-related user device at least partially based on the determined display optimization information;
wherein the display optimization information includes font brightness, font contrast, font-to-screen ratio information, screen brightness information, and color correction information; and
wherein the display setting adjustment unit acquires user device information, external environment information, and user preference information, and determines content display setting values based on the user device information, the external environment information, and the user preference information.

12. The user device of claim 11, further comprising a camera unit configured to acquire photographed ocular data of the user.

13. The user device of claim 11, further comprising at least one display unit.
